# DEMANDE DE BREVET EUROPEEN

(11) **EP 0 797 981 A1**
(43) Date de publication de la demande: **01.10.1997**
(21) Numéro de dépôt: 96120631.5
(22) Date de dépôt: 10.12.1993
(51) Int. Cl.: A61K 7/48, A61K 35/78, C12N 5/00, A61L 27/00

(54) **Utilisation d'un extrait de simarouba pour la fabrication d'un milieu de culture de cellules de peau**

(30) Priorité: 11.12.1992 FR 9214968; 02.08.1993 FR 9309492
(62) Demande divisionnaire de: 94902016.8
(71) Demandeur: LVMH RECHERCHE, 92752 Nanterre (FR)
(72) Inventeur: Bonte, Frédéric, 92400 Courbevoie (FR); Meybeck, Alain, 92400 Courbevoie (FR); Dumas, Marc, 92700 Colombes (FR)
(74) Mandataire: Portal, Gérard

(57) **Abrégé**

L'invention concerne l'utilisation d'un extrait de Simarouba.

Selon l'invention, on utilise un extrait de Simarouba pour la préparation d'un milieu de culture de cellules de peau. L'invention couvre aussi le milieu de culture.

L'extrait de Simarouba présente une activité de différenciation des kératinocytes significative permettant une culture de cellules de peau et d'obtention de peau artificielle.

## Description

La présente invention concerne essentiellement l'utilisation d'un extrait de Simarouba pour la préparation d'un milieu du culture de cellules de peau, et un milieu de culture.

Plus particulièrement l'invention est relative à l'utilisation d'un extrait de Simarouba pour la préparation d'un milieu de culture de cellules ou de tissus, notamment pour la culture en masse de cellules de peau, en particulier de kératinocytes.

La plante Simarouba est une plante de la famille des Simaroubacées. Parmi les plantes Simarouba, on peut citer Simarouba amara Aubl., Simarouba glauca, Simarouba versicolor et Simarouba excelsa. Ces plantes sont connues pour avoir des propriétés similaires (voir le livre de Mrs M. GRIEVE ayant pour titre "Modem Herbal" édité par Mrs C. F. LEYEL aux éditions PENGUIN BOOKS, pages 741-742). Simarouba amara Aubl., en particulier, est un arbre assez répandu en Guyane, dont l'écorce est utilisée par les indigènes comme remède pour traiter la dysenterie, améliorer la tonicité de l'intestin, promouvoir les sécrétions de l'intestin et prédisposer le patient à dormir.

D'autre part, l'abrégé Chemical Abstracts 110 (02) 013 432 décrit l'utilisation d'extrait de tige de Simarouba amara pour la mise en évidence in vitro d'une activité antiamibienne. De même, la revue Chemical Abstracts, résumé 109 (101) 000285 décrit un extrait de fruit de Simarouba amara pour la préparation d'un médicament anti-paludéen.

Or, les inventeurs de la présente invention ont pu observer que les extraits de Simarouba permettaient de favoriser, d'accélérer et d'améliorer la différenciation des cellules de peau, en particulier des kératinocytes, lors de leur culture dans un milieu de culture.

La présente invention a pour but principal de résoudre le nouveau problème technique consistant en la fourniture d'une solution permettant de favoriser, d'accélérer et d'améliorer la différenciation de cellules de peau, notamment de kératinocytes, en culture.

La présente invention résout pour la première fois ce problème technique de manière satisfaisante et utilisable à l'échelle industrielle pour la préparation de milieux de culture notamment en masse de cellules de peau.

Ainsi, selon un premier aspect, la présente invention concerne l'utilisation d'un extrait de Simarouba pour la préparation d'un milieu de culture de cellules ou de tissus, notamment pour la culture en masse de cellules de peau, en particulier de kératinocytes.

Selon une caractéristique particulière, l'extrait précité est obtenu à partir de Simarouba amara (Aublet), Simarouba glauca, Simarouba versicolor ou Simarouba excelsa, notamment à partir des écorces de troncs, de tiges ou de racines de ces plantes.

Selon une autre caractéristique particulière, l'extrait précité est un extrait obtenu par extraction avec au moins un solvant polaire, tel que l'eau, un alcool, de préférence un alcool inférieur, tel que méthanol ou éthanol, un glycol, en particulier le propylèneglycol, ou un mélange hydro-alcoolique en toute proportion.

Selon une autre caractéristique particulière, l'extrait précité est présent dans la composition à une concentration comprise entre 0,001 et 5 % en poids, exprimée en extrait sec, par rapport au poids total de la composition, de préférence entre 0,01 et 1 % et encore de préférence entre 0,1 et 0,5 % en poids.

Selon une autre caractéristique particulière, la composition précitée contient, en outre, un agent actif choisi parmi le groupe constitué par l'acide kojique et ses sels ou esters, l'acide caféique et ses sels ou esters, l'hydroquinone et ses dérivés, un extrait de mûrier, la trétinoïne, la vitamine A ou ses dérivés ou esters, un caroténoïde, notamment le bêta-carotène, la vitamine C, un corticoïde, le glutathion, la cystéine, l'acide parahydroxycinnamique ou ses sels ou esters, les sels, esters et dérivés des substances précitées étant choisis parmi ceux cosmétiquement ou pharmaceutiquement acceptables.

Selon encore une autre caractéristique particulière, la composition précitée peut en outre contenir au moins un agent hydratant, tel que l'acide hyaluronique.

Selon une autre caractéristique particulière, l'extrait de mûrier précité est présent en une proportion en poids comprise entre 0,001 et 5 %, de préférence entre 0,01 et 1 %, encore de préférence entre 0,1 et 0,8 % en poids par rapport au poids total de la composition.

Selon encore une autre caractéristique particulière, l'acide kojique ou ses sels ou esters précités est présent dis la composition à une proportion en poids comprise entre 0,001 et 5 %, encore de préférence entre 0,01 et 2%, en poids par rapport au poids total de la composition.

Selon une autre caractéristique particulière, l'extrait précité de Simarouba est au moins en partie incorporé dans des phases lamellaires lipidiques hydratées ou dans des liposomes, soit seul, soit combiné à au moins une autre substance active présente dans la composition.

La préparation des liposomes contenant au moins en partie l'extrait de Simarouba précité peut être réalisée selon l'un des procédés connus pour incorporer des substances actives dans des liposomes.

Selon un mode de réalisation préféré, selon la présente invention, on utilise un procédé d'atomisation des constituants de la phase lipidique, permettant d'obtenir une poudre lipidique facilement dispersible dans une solution aqueuse pour former des liposomes par exemple selon le procédé décrit dans le document US-A-4 508 703. La suspension de liposomes ainsi obtenue peut être homogénéisée au moyen d'ultrasons, ou dans le cas d'une production de masse, au moyen d'une homogénéisation sous pression, conformément au procédé décrit dans US-A-4 621 023.

L'extrait de Simarouba précité peut être incorporé soit dans la phase lipidique soit dans la phase aqueuse des liposomes en particuclier en utilisant les procédés précédemment décrits. Dans le cas de l'incorporation dans la phase lipidique des liposomes, on peut procéder comme suit. On dissout l'extrait de Simarouba avec les constituants de la phase lipidique, avant l'atomisation, dans une solution organique contenant au moins un lipide amphiphile, tel que la lécithine de soja, éventuellement un composé hydrophobe liphophile tel qu'un stérol, en particulier le cholestérol ou le β-sitostérol. De préférence, le solvant est choisi parmi le dichlorométhane, le chloroforme ou le méthanol, ou l'un de leurs mélanges.

La solution organique peut avantageusement contenir un agent antioxydant tel que l'α-tocophérol.

La poudre lipidique obtenue est dispersée dans un milieu aqueux convenable, par exemple une solution tampon PBS, une solution de glucose ou une solution de chlorure de sodium. On obtient ainsi une suspension de liposomes.

Selon un mode de réalisation avantageux, et surtout dans le cas d'une composition de liposomes, après avoir éventuellement homogénéisé la composition obtenue, les compositions de liposomes sont gélifiées par mélange avec un gel, tel qu'un gel de polymère vinylique, en particulier commercialisé sous la dénomination commerciale Carbopol® 940. Cette procédure de gélification est également décrite dans US-A-4 508 703, en particulier dans les exemples.

Selon un mode de réalisation avantageux de l'invention, la concentration de l'extrait de Simarouba précité est comprise entre 0,001 et 30% en poids, de préférence entre 0,01 et 10% en poids, de la phase lipidique desdits liposomes.

Selon un deuxième aspect, la présente invention concerne encore un milieu de culture de cellules ou de tissus, notamment pour la culture de cellules de peau, en particulier de kératinocytes, permettant de favoriser, accélérer et améliorer leur différenciation, caractérisé en ce qu'il comprend une quantité efficace d'un extrait de Simarouba pour obtenir une telle différenciation.

Selon un troisième aspect, l'invention concerne encore un procédé pour favoriser, accélérer ou améliorer la différenciation des cellules de peau, en particulier des kératinocytes, notamment dans le cadre d'une culture en masse de cellules de peau, pour la production de peau artificielle ou pour la préparation de modèles de peau reconstituée, caractérisé en ce que l'on utilise un milieu de culture tel que défini dans la description précédente, ou dans la description suivante prise dans son ensemble.

Le procédé selon l'invention dit de différenciation cellulaire présente notamment un grand intérêt industriel. On citera par exemple :
- la production de médiateurs biochimiques par traitement de culture en masse de kératinocytes dans des bioréacteurs;
- la préparation de peau artificielle en vue de réaliser des greffes de peau, le procédé de l'invention étant particulièrement avantageux dans le cas d'auto-greffes de grands brûlés, grâce au gain de temps qu'il procure dans la préparation d'une peau artificielle. En particulier, l'accélération et l'amélioration de la différenciation des kératinocytes se traduit par la formation plus rapide d'une couche comée, de bonne qualité;
- la réalisation de modèles de peau comprenant de la peau reconstituée, destinée par exemple à évaluer la pénétration cutanée ou la toxicité d'une substance ou composition appliquée par voie topique, lorsqu'elle est destinée en particulier à un traitement local ou à un traitement systémique (transderme), notamment.

Selon une variante de réalisation particulière, ce procédé de culture comprendra généralement la préparation d'un milieu de culture pour la croissance de kératinocytes humains comprenant un milieu de base nutritif DMEM (Gibco®), un facteur de croissance épidermique ("EGF"), 10% de sérum de veau foetal, de l'isoprotérénol et/ou de la forskoline, ainsi que de l'hydrocortisone. Dans le cadre de l'invention, ce milieu comprend aussi un extrait de Simarouba tel que décrit dans la description précédente ou suivante, généralement à une concentration de 0,01 à 0,5% en poids.

Dans ce procédé, on réalise une culture en masse de cellules de peau en inoculant des kératinocytes afin de les immobiliser sur des supports tels que fibres creuses, des microbilles, ou des matrices microporeuses et cela à l'aide du milieu de culture ci-dessus. On peut assurer une perfusion du milieu afin d'avoir un apport suffisant à la croissance et la différenciation même lorsque la biomasse est importante.

Le milieu de culture selon l'invention peut avantageusement être utilisé pour la culture en masse de cellules de peau, en particulier de kératinocytes, pour la production de peau artificielle ou pour la préparation de modèles de peau reconstituée.

De préférence, dans les différents aspects précédents de l'invention, selon une variante de réalisation particulière, on peut utiliser de 0,01 à 0,5% en poids d'extrait de Simarouba, par rapport au poids total du milieu de culture final.

Ainsi, d'autres buts, caractéristiques et avantages de l'invention apparaîtront clairement à la lumière de la description explicative ci-après faite en référence à plusieurs exemples illustratifs de l'invention qui ne Sauraient donc en aucune façon limiter la portée de l'invention. Dans la présente description, incluant les exemples, les pourcentages sont donnés en poids sauf indication contraire.

### Exemple 1 de l'invention

### Extrait aqueux d'écorce de Simarouba

A partir d'un extrait d'écorce de racines de l'arbre Simarouba amara Aubl. en provenance de Guyane, on réalise une extraction avec de l'eau par extraction de type Soxhlet, c'est-à-dire au reflux pendant plusieurs heures. Le rapport solvant-écorce est en général de 10-1 en poids.

Après extraction, généralement on concentre cet extrait en vue d'une utlisation cosmétique ou pharmaceutique. L'extrait concentré est dénommé produit I1.

Naturellement, comme cela est bien compréhensible à l'homme de l'art, on peut poursuivre l'élimination du solvant, ici l'eau, jusqu'à obtention d'un extrait sec, par évaporation sous pression réduite ou par lyophilisation.

### Exemple 2 de l'invention

### Extrait méthanolique d'écorce de racines de Simarouba

A partir de 100 g d'écorce de racines de l'arbre Simarouba amara (Aubl.) en provenance de Guyane, on réalise une extraction avec 1 l de méthanol selon la méthode dite de Soxhlet, pendant plusieurs heures à reflux.

Après extraction, l'extrait méthanolique est concentré jusqu'à obtention d'un produit contenant très peu de méthanol dénommé produit I2.

### Test d'activité d'extrait de Simarouba sur la différenciation des kératinocytes

Pour la présente étude, on utilise un prélèvement de peau effectué lors d'une opération de chirurgie esthétique de type "lifting" sur le visage d'une femme de 61 ans.

Au jour J=-2, on ensemence 12 puits d'une plaque de culture multipuits avec 10⁵ cellules par puits de kératinocytes isolés à partir de ce prélèvement dans un milieu de culture classique pour la culture des kératinocytes, bien connu de l'homme de l'art. Avantageusement, ce milieu de culture peut être constitué par un milieu M199 additionné de 2 mM de L-glutamine, de 10% de sérum de veau foetal, de toxine cholérique, d'hydrocortisone, ainsi que du facteur de croissance épidermique EGF.

Aux jours J=0,J=4,J=7 et J=10, le milieu de culture est renouvelé par un milieu identique sur 6 des 12 puits qui constituent des puits témoins non traités, et, sur les 6 puits restants, constituant les puits traités on renouvèle avec un milieu identique, mais dans lequel on a dissous 25 µg/ml d'extrait de Simarouba tel qu'obtenu à l'exemple 1, et sur lesquels on réalise une filtration stérilisante sur filtre, par exemple à 0,22 µm.

Au jour J=12, on réalise une numération cellulaire ainsi qu'une inclusion des cellules en résine époxy après fixation et marquage des structures cellulaires à l'acétate d'uranyle.

On réalise une coupe de chacun des blocs de résine ainsi obtenus sous forme de coupe ultrafine, par exemple à l'aide d'un dispositif connu sous le nom de Microtome, et on observe celle-ci au microscope électronique à transmission.

On a ensuite observé chacune des 6 coupes de la culture témoin, d'une part, et des 6 coupes de la culture traitée par l'extrait de Simarouba, d'autre part.

On a pu observer que les cellules traitées avec l'extrait de Simarouba selon l'invention, par exemple l'extrait obtenu à l'exemple 1, présentent une différenciation cellulaire nettement supérieure au témoin, à Savoir :
- plus de 10 couches cellulaires alors que le témoin présente 5 couches cellulaires en moyenne et au maximum 8;
- 3 à 4 couches intermédiaires composées de kératinocytes ayant une abondante activité cytoplasmique, des desmosomes et des tonofilaments nombreux et réguliers, tandis que la culture témoin présente des cellules n'ayant pas ces caractéristiques, et possède une architecture non régulière;
- les couches supérieures présentent une cornification régulière avec plusieurs couches de cornéocytes présentant une enveloppe cornée épaisse et remplie de tonofilaments, tandis que les couches supérieures des témoins possèdent peu de cornéocytes et présentent donc une cornéification très incomplète, une absence de grains de kératohyaline, la présence de restes d'organelles cellulaires, preuves d'une mauvaise différenciation.

Ces résultats nettement supérieurs sur la différenciation cellulaire obtenue avec l'extrait de Simarouba selon l'invention permet d'envisager l'application des extraits de Simarouba à la préparation de composition cosmétique ou pharmaceutique, notamment dermatologique, en particulier suivante :
- des compositions favorisant la formation d'un épiderme bien différencié donc donnant une peau "belle" ayant un grain et un toucher agréables;
- des compositions luttant contre les troubles de la différenciation épidermique, survenant notamment au cours du vieillissement;
- des compositions renforçant la barrière cutanée donc protégeant des agressions extemes, par exemple par des allergènes ou des tensioactifs, d'une part, et limitant la perte excessive d'eau par l'épiderme, d'autre part;
- des compositions permettant d'améliorer la qualité de la chevelure notamment par une amélioration de la qualité de la kératine de la tige pilaire du cheveu provenant de l'activité des kératinocytes des follicules qui se différencient depuis la base du poil.

Egalement, on peut envisager l'application de ces extraits à la préparation de milieux de cultures cellulaires, en particulier destinés à la culture en masse de kératinocytes, ou de compositions destinées à traiter des cultures de kératinocytes pour favoriser la vitesse et/ou la qualité de l'architecture de ces cultures lorsqu'elles sont, en particulier, destinées à fabriquer des peaux artificielles, dites équivalentes pour greffes, en particulier sur des brûlés, ou bien pour la préparation de modèles destinés à évaluer la toxicité ou la pénétration cutanée de diverses substances testées.

On donne ci-après un exemple de milieu de culture selon l'invention, utilisant un extrait de Simarouba.

### Exemple 4

### Préparation d'un milieu de culture et son utilisation pour la culture en masse de cellules de peau

Pour la préparation de ce milieu de culture ainsi que pour son utilisation pour la culture en masse de cellules de peau, l'homme de l'art pourra se reporter notamment à l'article de Philip C. Familletti et al. dans Biotechnology, vol. 6, janvier 1988, pages 41 à 44 ainsi qu'aux références citées dans cet article, en particulier Arathoon et al. dans la revue Science (1986), 232, page 1390 et suivantes; l'article de Ku, K. et al. dans la revue Biotechnology, Bioeng. (1980), 23, pages 79 et suivantes.

Ainsi, on prépare un milieu de culture optimal pour la croissance de kératinocytes humains comprenant un milieu de base nutritif DMEM (Gibco®), du facteur de croissance EGF, 10% de sérum de veau foetal, de l'isoprotérénol et/ou de la forskoline, ainsi que de l'hydrocortisone.

Ce milieu comprendra un extrait de Simarouba selon l'invention, de préférence à la concentration de 0,01 à 0,5 % en poids d'extrait de Simarouba par rapport au poids total du milieu de culture final.

On réalise une culture à base de cellules de peau en inoculant des kératinocytes afin de les immobiliser sur des supports tels que fibres creuses, des microbilles, ou des matrices microporeuses et cela à l'aide du milieu de culture décrit précédemment. Une perfusion en milieu dans un système à perfusion du type de celui-ci décrit par Sylvie GUICHARD-BALESTRINI dans la revue Biofutur, supplément n°56, avril 1987, pages 2 à 14, sera assurée afin d'avoir un apport suffisant à la croissance et à la différenciation même lorsque la biomasse est importante.

En fin de culture, on récupère les substances sécrétées par les kératinocytes, contenant principalement des lipides, sources de matière première pour la formulation de compositions cosmétiques ou pharmaceutiques à application topique sur l'épiderme ou le cuir chevelu.

Grâce au produit de l'invention, il est possible de traiter des cultures de peau reconstituées, en particulier des cultures de kératinocytes ou d'autres cellules épidermiques cultivées sur un support approprié, tel qu'un support collagénique (contenant ou non des fibroblastes), par exemple décrit dans le document La Recherche, (1987), n°185, pages 149-159 et dans Br. J. Dermatol. (1986), 114, pages 91-101, ou un support constitué par du derme excisé.

Grâce à l'utilisation de la composition selon l'invention, on obtiendra une épidermisation plus rapide et plus complète. Ceci permettra dans des délais plus courts de fournir aux médecins de la peau reconstituée, une sorte de pansement biologique pour les autogreffes, par exemple dans le cas de brûlures étendues. L'invention permettra également de réaliser de façon industrielle et concurrentielle des peaux reconstituées de bonne qualité pour réaliser des tests de pénétration ou de tolérance.

L'invention comprend naturellement tous les moyens constituants des équivalents techniques des moyens décrits ainsi que leurs diverses combinaisons.

L'invention couvre également toute caractéristique qui apparaît être nouvelle vis-à-vis d'un état de la technique quelconque, à partir de la présente description prise dans son ensemble.

## Revendications

1. Utilisation d'un extrait de Simarouba pour la préparation d'un milieu de culture de cellules ou de tissus, notamment pour la culture en masse de cellules de peau, en particulier de kératinocytes.

2. Utilisation selon la revendication 1, caractérisée en ce que l'extrait précité est obtenu à partir de Simarouba amara (Aublet), Simarouba glauca, Simarouba versicolor ou Simarouba excelsa, notamment à partir des écorces de troncs, de tiges ou de racines de ces plantes.

3. Utilisation selon la revendication 1 ou 2, caractérisé en ce que l'extrait précité est un extrait obtenu par extraction avec au moins un solvant polaire, tel que l'eau, un alcool, de préférence un alcool inférieur, tel que méthanol ou éthanol, un glycol, en particulier le propylèneglycol, ou un mélange hydroalcoolique en toute proportion.

4. Utilisation selon l'une des revendications 1 à 3, caractérisée en ce que l'extrait précité est présent dans la composition à une concentration comprise entre 0,001 et 5 % en poids, exprimée en extrait sec, par rapport au poids total de la composition, de préférence entre 0,01 et 1 % et encore de préférence entre 0,1 et 0,5% en poids.

5. Utilisation selon l'une des revendications précédentes, caractérisée en ce que la composition précitée contient, en outre, un agent actif choisi parmi le groupe constitué par l'acide kojique et ses sels ou esters, l'acide caféique et ses sels ou esters, l'hydroquinone et ses dérivés, un extrait de mûrier, la trétinoïne, la vitamine A ou ses dérivés ou esters, un caroténoïde, notamment le bêta-carotène, la vitamine C, un corticoïde, le glutathion, la cystéine, l'acide parahydroxycinnamique ou ses sels ou esters, les sels, esters et dérivés des substances précitées étant choisis parmi ceux cosmétiquement ou pharmaceutiquement acceptables.

6. Utilisation selon l'une des revendications précédentes, caractérisée en ce que la composition précitée peut en outre contenir au moins un agent hydratant, tel que l'acide hyaluronique.

7. Utilisation selon la revendication 5 ou 6, caractérisée en ce que l'extrait de mûrier précité est présent en une proportion en poids comprise entre 0,001 et 5 %, de préférence entre 0,01 et 1 %, encore de préférence entre 0,1 et 0,8 % en poids par rapport au poids total de la composition.

8. Utilisation selon la revendication 5 ou 6, caractérisée en ce que l'acide kojique ou ses sels ou esters précités est présent dans la composition à une proportion en poids comprise entre 0,001 et 5 %, encore de préférence entre 0,01 et 2 %, en poids par rapport au poids total de la composition.

9. Utilisation selon l'une des revendications précédentes, caractérisée en ce que l'extrait précité de Simarouba est au moins en partie incorporé dans des phases lamellaires lipidiques hydratées ou dans des liposomes, soit seul, soit combiné à au moins une autre substance active présente dans la composition.

10. Utilisation selon la revendication 9, caractérisée en ce que la concentration de l'extrait de Simarouba précité est comprise entre 0,001 et 30% en poids, de préférence entre 0,01 et 10% en poids, de la phase lipidique desdits liposomes.

11. Milieu de culture de cellules ou de tissus, notamment pour la culture de cellules de peau, en particulier de kératinocytes, permettant de favoriser, d'accélérer et d'améliorer leur différenciation, caractérisé en ce qu'il comprend une quantité efficace d'un extrait de Simarouba pour obtenir une telle différenciation.

12. Milieu de culture selon la revendication 11, caractérisé en ce qu'il comprend de 0,01 à 0,5% en poids d'extrait de Simarouba par rapport au poids total du milieu de culture final.

13. Milieu de culture selon la revendication 11 ou 12, caractérisé en ce qu'il est utilisé pour la culture en masse de cellules de peau, en particulier de kératinocytes, pour la production de peau artificielle ou pour la préparation de modèles de peau reconstituée.

14. Procédé pour favoriser, accélérer ou améliorer la différenciation des cellules de peau, en particulier des kératinocytes, notamment dans le cadre d'une culture en masse de cellules de peau, pour la production de peau artificielle ou pour la préparation de modèles de peau reconstituée, caractérisé en ce qu'on utilise un milieu de culture tel que défini dans l'une des revendications 11 à 13.

15. Procédé selon la revendication 14, caractérisé en ce qu'on prépare un milieu de culture pour la croissance de kératinocytes humains comprenant un milieu de base nutritif DMEM, un facteur de croissance épidermique EGF, 10% de sérum de veau foetal, de l'isoprotérénol et/ou de la forskoline, ainsi que de l'hydrocortisone.

16. Procédé de culture selon la revendication 14 ou 15, caractérisé en ce qu'on réalise une culture en masse de cellules de peau en inoculant des kératinocytes afin de les immobiliser sur des supports tels que fibres creuses, des microbilles, ou des matrices microporeuses, éventuellement en assurant une perfusion du milieu afin d'avoir un apport suffisant à la croissance et la différenciation même lorsque la biomasse est importante.
